# EUROPEAN PATENT SPECIFICATION

(11) **EP 2 983 678 B1**
(45) Date of publication and mention of the grant of the patent: **02.01.2019**
(21) Application number: 14783051.7
(22) Date of filing: 10.02.2014
(51) Int. Cl.: A61K 31/722, A61P 31/04, A61P 29/00, A61K 45/06

(54) **ANTI-MICROBIAL COMPOSITION**
ANTIMIKROBIELLE ZUSAMMENSETZUNG
COMPOSITION ANTIMICROBIENNE

(30) Priority: 10.04.2013 SG 201302687
(43) Date of publication of application: 17.02.2016
(73) Proprietor: Areteon Pte Ltd, Singapore 415979 (SG)
(72) Inventor: CHUA, Eng Kheng Alfred, Singapore 415979 (SG); LOW, Soo Chee, Singapore 415979 (SG)
(74) Representative: Lehmann Novo, Maria Isabel
(86) International application number: PCT/SG2014/000053
(87) International publication number: WO 2014/168579

(56) References cited:
- WO-A1-2011/007454
- WO-A2-2010/051151
- DATABASE WPI Week 200551 Thomson Scientific, London, GB; AN 2005-498442 XP002769455, -& CN 1 583 990 A (UNIV WUHAN) 23 February 2005 (2005-02-23)
- RICHARDSON K. ET AL.: 'Physicochemical and antibacterial properties of surfactant mixtures with quatemized chitosan microgels' CARBOHYDRATE POLYMERS vol. 93, no. 2, 01 January 2013, pages 709 - 717, XP028996615 DOI: 10.1016/J.CARBPOL.2012.12.054
- LI P. ET AL.: 'A polycationic antimicrobial and biocompatible hydrogel with microbe membrane suctioning ability' NATURE MATERIALS vol. 10, 2011, pages 149 - 156, XP055293738

## Description

### FIELD

The present invention relates to a topical anti-microbial composition and method of use thereof. In particular, the present invention relates to a topical anti-microbial composition comprising a quaternized chitosan derivative having a polymerizable organic moiety and an amphoteric surfactant.

### BACKGROUND

The treatment of skin conditions such as dermatitis, which often involve bacterial infection, remains difficult. Dermatitis including external acoustic meatus inflammation, dermatitis, eczema, dermatomycosis, pyoderma, allergic dermatitis, urtication, traumatic dermatitis and alopecia are all diseases with the potential for bacterial infection that might require anti-microbial agents. Pyoderma, in particular, typically involves bacterial infections leading to impetigo, impetigo contagiosa, ecthyma, folliculitis, Bockhart's impetigo, furuncle, carbuncle and tropical ulcer. Even superficial pyoderma is typically associated with bacterial infection that involves the epidermis and portions of the hair follicle near the surface of the skin.

Dermatological conditions which include bacterial infection are most often treated using topical anti-microbial compositions. However, it is well documented that in recent times numerous bacteria have become resistant to particular antibiotics due to their overuse. Accordingly, there is a continuing need for the development of new anti-microbial compositions for the treatment of bacterial infections particularly in conditions such as dermatitis, which are not antibiotic based.

Recently it has been shown that chitosan, a [beta]-1,4-linked polymer of glucosamine (2-amino-2-deoxy-[beta]-D-glucose), has anti-microbial properties. In particular, quaternized chitosans have found utility as anti-microbial agents in disinfectants (JP-A-2005-290297, JP-A-8-144121, JP-A-2004-515813). Thus, these agents might be useful as a replacement of, or supplement to, traditional antibiotic treatments for skin conditions.

### SUMMARY

The inventor has surprisingly found that a topical anti-microbial composition comprising an effective amount of a quaternized chitosan derivative having a polymerizable organic moiety and an amphoteric surfactant has a synergistic anti-microbial action in the treatment of bacterial infections.

Thus, the present invention provides a topical anti-microbial composition and a method of use and especially provides a topical anti-microbial composition which is useful when applied to the skin and/or hair or fur of mammals as washes and shampoos.

Accordingly, in a first aspect the present invention provides a topical anti-microbial composition comprising a quaternized chitosan derivative having a polymerizable organic moiety and an amphoteric surfactant.

In a second aspect the present invention provides a topical anti-microbial composition consisting essentially of a quaternized chitosan derivative having a polymerizable organic moiety and an amphoteric surfactant.

Formulations of the topical anti-microbial composition of this invention may further include additional anti-microbial agents, skin softening and moisturizing agents, as well as skin cleansing agents. As an option, dyes or fragrances may be added for desired cosmetic properties. The topical anti-microbial composition may also be mixed with conventionally acceptable adjuvants, excipients, stabilizers, diluents, anti-foaming agents and/or extenders usable in the art.

In some embodiments, the topical composition of the present invention comprises from about 1% to about 10% of a stabilizer selected from the group consisting of glyceryl monostearate, stearic acid, triethanolamne, ethanol, polysorbate 20, cetyl alcohol, stearyl alcohol, cetrimonium bromide, citric acid, cyclomethicone, dimethicone, ceteth 20, ceteareth 20, caprylic/capric triglycerides, PEG 40 polyhydroxystearate, polyvinyl pyrrolidone, acetum, glyceryl stearate, xanthan gum, geranium oil, lavender oil, eucalyptus oil, tea tree oil, lemon oil, anise oil, DEA cetyl phosphate, sodium stearate, potassium stearate, wool alcohols, octyl stearate, carnauba wax, ozokerite, carbomer, phenoxyethanol, methyl parabens and propyl parabens and mixtures thereof

In some embodiments, the concentration of the quaternized chitosan derivative that is between about 1%:w/v and about 10% w/v, more preferably between about 2% w/v and 6% w/v and even more preferably between about 2% w/v and 4% w/v and typically less than or about 4%w/v.

In a third aspect, the present invention provides a topical anti-microbial composition comprising a quaternized chitosan derivative having polymerizable organic moiety and an amphoteric surfactant for use as a medicament for the treatment of skin infections in animals.

In a fourth aspect; the present invention provides a method of forming the topical anti-microbial composition of the first, second or third aspects comprising the step of mixing at least one quaternized chitosan derivative having a polymerizable organic moiety with at least one amphoteric surfactant.

In a fifth aspect, the present invention provides a topical composition according to the first, second or third aspects for use in a method of treating skin infections in mammals comprising the step of topically applying to said skin an effective amount of the topical anti-microbial composition.

In a seventh aspect, the present invention provides a topical composition according to first, second or third aspects for use in a method for treating or preventing a microbial skin infection in a mammal, comprising the step of applying to said mammal the topical anti-microbial composition.

In some embodiments, the topical anti-microbial composition is applied to infected skin of said mammal.

In an eighth aspect the present invention provides the use of a topical anti-microbial composition comprising a quaternized chitosan derivative having a polymerizable organic moiety and an amphoteric surfactant in the manufacture of a medicated shampoo for the treatment of a microbial skin infection in mammals.

The topical anti-microbial composition of the present invention is effective against a large range of microorganisms; however, it is especially suited for the treatment of bacterial infections caused by *Staphylococcus* species and *Escherichia* species, in particular *Staphylococcus aureus, Staphylococcus pseudintermedius,* and *Escherichia coli.*

It will be appreciated by those skilled in the art that the topical anti-microbial composition can be applied to any mammal which is likely to come into contact with microbes.

### DETAILED DESCRIPTION OF THE PREFERRED EMBODIMENTS

It is to be understood that this disclosure is not limited to particularly exemplified methods and may, of course, vary. It is also to be understood that the terminology used herein is for the purpose of describing particular embodiments only, and is not intended to be limiting which will be limited only by the appended claims.

In this specification and in the claims that follow, reference will be made to a number of terms that shall be defined to have the following meanings:
The term "comprising" is meant including, but not limited to, whatever follows the word "comprising". Thus, use of the term "comprising" indicates that the listed elements are required or mandatory, but that other elements are optional and may or may not be present. By "consisting of' is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that other elements are optional and may or may not be present depending upon whether or not they affect the activity or action of the listed elements.

It must be noted that, as used in the specification and the appended claims, the singular forms "a," "an" and "the" include plural referents unless the context clearly dictates otherwise. Thus, for example, reference to "a composition" includes mixtures of two or more such compositions, and the like.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance can or cannot occur, and that the description includes instances where the event or circumstance occurs and instances where it does not.

A weight percent of a component, unless specifically stated to the contrary, is based on the total weight of the formulation or composition in which the component is included.

In the broadest aspect of the present invention there is provided a topical anti-microbial composition comprising a quaternized chitosan derivative having polymerizable organic moieties and an amphoteric surfactant.

An "anti-microbial composition" refers to composition that has biological activity. The anti-microbial composition can be used to treat, cure, mitigate, prevent (i.e., prophylactically), ameliorate, modulate, or have an otherwise favourable effect on a microbial infection of skin.

The term "quaternized chitosan derivative" as used herein refers to water-soluble derivatives of chitosan that have polymerizable organic moieties. In one embodiment, the "quaternized chitosan derivative" has an ammonium group represented by formula (1):

-N⁺(R¹)₃

where each R¹ is independently selected from the group consisting of hydrogen and a substituted or unsubstituted alkyl group, two or three of R¹s may combine to form a substituted or unsubstituted aliphatic cyclic group.

In one embodiment, at least one of R¹s is a substituted or unsubstituted alkyl group having 1 to 18 carbon atoms.

Preferably, the polymerizable organic moiety of the quaternized chitosan derivative is represented by formula (2):

-Y-P

where P is a polymerizable group; Y is a spacer group.

In some embodiments, the quaternized chitosan derivative the -N⁺(R¹)₃ group is

-N⁺R'R"₂

where R' and R" are independently selected from the group consisting of hydrogen and a substituted or unsubstituted alkyl group, provided that R' has more carbon atoms than R".

A schematic structure of the quaternized chitosan derivative of the present invention is shown in formula (3): 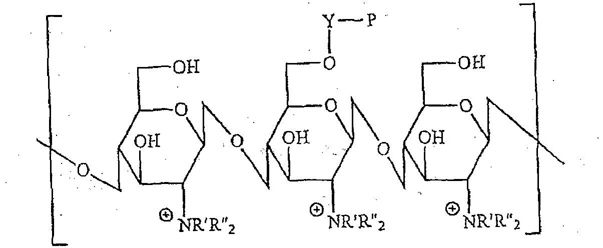 where P is a polymerizable group; Y is a spacer group; and R' and R" are independently selected from the group consisting of hydrogen and a substituted or unsubstituted alkyl group. Formula (3) is for only illustrative purposes only and is not intended to be restrictive on the present invention.

In one embodiment the quaternized chitosan derivative is a cationic quaternized chitosan derivative. The cationic quaternized chitosan derivative contains trialkylammonium groups along the quaternized chitosan chain. The quaternized chitosan will also contain a polymerizable group attached to the quaternized chitosan chain via a primary hydroxyl group, preferably via a spacer group Y.

Referring to formula (3), preferred embodiments of the cationic quaternized chitosan derivative will comprise a quaternized chitosan containing trialkylammonium groups along the polymer chain, wherein all three alkyl groups are identical. Non-limiting examples of the quaternized ammonium moiety include trimethylammonium-, triethylammonium-, tripropylammonium-, tributylammonium-, tripentylammonium-, trihexylammonium-, trioctylammonium-, tridecylammonium-, tridodecylammonium-, trioctadecylammomium-.

In some embodiments, the preferred trialkylammonium moieties comprise one alkyl group that differs from the other two. Non-limiting examples include dimethylethylammonium-, dimethylpropylammonium-, dimethylbutylammonium-, dimethylpentylammonium-, dimethylhexylammonium-, dimethyloctylammonium-, dimethyldecylammonium-, dimethyldodecylammonium-, dimethyloctadecylammomium-dihexadecylmethylammonium-, dihexadecylethylammonium-, dihexadecylpropylammonium-, and dihexadecylbutylammonium-.

Again, referring to formula (3), the polymerizable group P may comprise one of the polymerizable groups selected from the group comprising vinyl, acrylate, methacrylate, vinyl phenylene, cinnamoyl, allyl. The polymerizable group P is preferably attached to the cationic quaternized chitosan via a spacer group Y. The spacer group may be selected from the group comprising -(C*ₙ*H*₂ₙ*)-, or more preferably -(C*ₙ*H*₂ₙ*O)-, wherein n=0 to 20, or more preferably between 2 and 10.

Representative examples of methods of synthesizing the quaternized chitosan derivatives of the present invention include the following:
1). Preparing chlorofunctionalised PEG Acrylate derivative can be achieved by dissolving PEG monoacrylate (2.36 ml, 7.06 mmol), with an average molecular weight of 375 in 100 ml toluene and treating same with chloroacetylchloride (2.25 ml, 28.24 mmol). The mixture can then be heated under reflux for 24 hours. After this time, the solvent and volatiles are removed by evaporation and the residue can be dissolved in methylene chloride (150 ml). The solution can then be stirred over potassium carbonate and filtered. The solvent is removed through evaporation and after washing with hexane, the product is obtained and dried.
2). The method for preparing trimethylammonium chitosan comprises the step of adding 1 g of chitosan to 50 ml N-methyl-2-pyrrolidinone and then treating said mixture with 1.5 N NaOH solution (15 ml). The mixture is then stirred for 30 min at 50°C. Sodium iodide (1.08 g) and methyl iodide (11.2 g) are then added to the solution, which is then stirred for 24 hours at 50°C. The reaction mixture is then filtered to remove the insoluble material, and the filtrate is then precipitated into a large excess of acetone and filtered. The resulting product is collected by filtration, and dried under vacuum.
3). The method for preparing trihexylammonium chitosan comprises the same method as 2) above except instead of methyl iodide (11.2 g), hexyl bromide (13.0 g) is used.
4). The method for preparing tridecylammonium chitosan comprises the same method as 2) above except instead of methyl iodide (11.2 g), decyl bromide (17.4 g) is used
5). The method for preparing N-hexyl chitosan comprises the step of mixing 1.0 g chitosan (6.21 mmol) with 1% aq. acetic acid (100 ml). Hexanal (0.62 g, 0.74 ml, 1 eqv.) was then added, and the mixture stirred at room temperature. After 1 hour of stirring, the pH of the solution was adjusted to 4.5. To this solution, 10% aq. solution of sodium borohydride (9.32 mmol) was added and the solution stirred for an additional 90 minutes. After this time; the pH of the solution was adjusted to 10, and the precipitated N-hexyl chitosan collected by filtration, and washed with water until the filtrate was of neutral pH. These precipitants were filtered and the residue washed with distilled water to neutrality. The unreacted aldehyde and inorganic products were soxhlet extracted with ethanol and diethyl ether. The resulting N-hexyl chitosan was filtered and dried.
6). The method for preparing N-decyl chitosan was the same method as 5) except instead of hexanal we used decanal (0.97 g, 6.2 mmol).
7). The method for preparing dimethylhexylammonium chitosan comprises N-Hexyl chitosan from 5) above was added to N-methyl-2-pyrrolidinone (50 ml) and treated with 1.5 N NaOH solution (15 ml). The mixture was stirred for 30 min at 50°C. Sodium iodide (1.08 g) and methyl iodide (11.2 g) were then added to the solution, which was then stirred for 24 hours at 50°C. The reaction mixture was then filtered to remove the insoluble material, and the filtrate was then precipitated into a large excess of acetone and filtered. The resulting product was collected by filtration, and dried under vacuum.
8). The method for preparing dimethyldecylammonium chitosan was the same as 7) except instead of N-hexyl chitosan (1 g) we used N-decyl chitosan (1 g, see Synthetic Example 6).

For greater detail on the preparation of quaternized chitosan derivatives of the present invention we refer to US2012/0164208.

In some embodiments, the specific quaternized chitosan used in the anti-microbial composition of the present invention is termed DMDC-Q-g-EM which stands for dimethyldecylammonium chitosan with a high degree of quaternization. The DMDC-Q-g-EM may be prepared by any method known in the art; however, one preferred method is as follows:

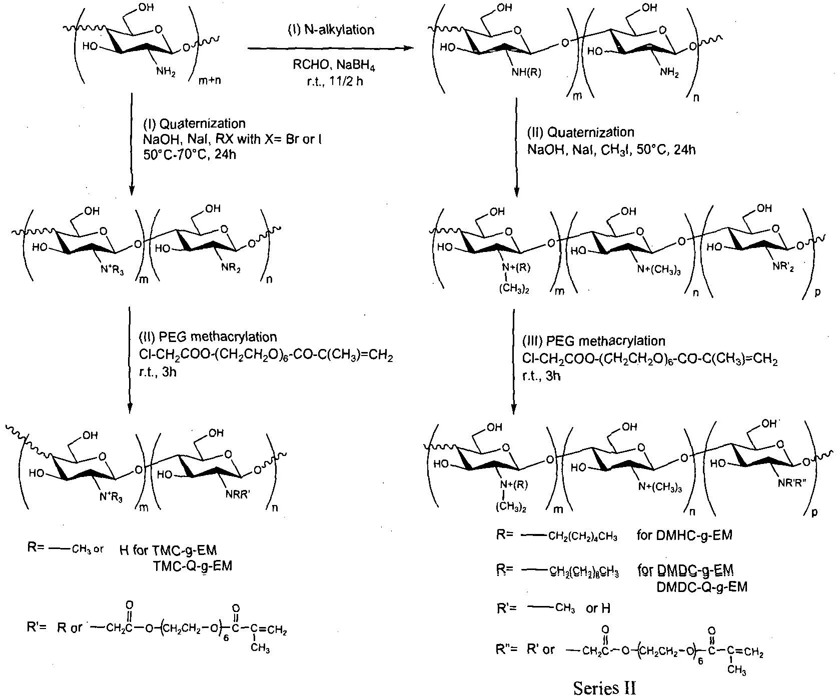

The methylation procedure to prepare dimethyldecylammonium chitosan (DMDC) uses chitosan (1 g, 6.21 mmol) in N-Methyl-2-pyrrolidone (NMP) (50 ml), which is added to NaOH solution (1.5M, 15 ml). Following incubation at 50°C, methylation was performed as follows by adding sodium iodide (1.08 g, 7.23 mmol) and methyl iodide (11.2 g, 78.7 mmol) to the chitosan/NMP/NaOH mixture and then reacted for 24 h at 50°C. The solution was then suction filtered. After dropping the filtrate into acetone (400ml), the precipitate obtained was filtered and then dried under vacuum to yield the product.

The term "amphoteric surfactants" as used herein refers to the following surfactants: N-alkylglycines, N-alkylaminopropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids, each having about 8 to 24 C atoms in the alkyl group, alkylaminoacetic acids each having about 8 to 24 C atoms in the alkyl group, N-cocoalkyl aminopropionate, cocoacyl aminoethyl aminopropionate, C₁₂-C₁₈ acyl sarcosine, N-alkyl-N,N-dimethylammonium glycinates, for example, cocoalkyl dimethylammonium glycinate, N-acyl-aminopropyl-N,N-dimethylammonium glycinates, for example, cocoacyl aminopropyl dimethylammonium glycinate, 2-alkyl-3-carboxymethyl-3-hydroxyethyl imidazolines each having about 8 to 18 C atoms in the alkyl or acyl group, cocoacyl aminoethyl hydroxyethyl carboxymethyl glycinate, compounds known under the INCI name cocamidopropyl betaine, compounds known under the INCI name disodium cocoamphodiacetate, with preferred agents containing amphoteric surfactant(s) in amounts of 1 to 15 wt. %, preferably 2.5 to 12 wt. % and particularly 5 to 10 wt. %, based on total weight of the agent.

In some embodiments, the specific concentration of the quaternized chitosan derivative used is between about 1% w/v (10,000 ppm) and about 10% w/v (100,000 ppm), more preferably between about 2% w/v and 6% w/v and even more preferably between about 2% w/v and 4% w/v and typically less than or about 4%w/v. However, as shown herein antibacterial effect can be achieved with as little as 20 ppm to 40 ppm i.e. 0.002% w/v to 0.004% w/v.

As used herein, the terms "anti-microbial composition" or "anti-microbial compositions," "anti-microbial," "anti-bacterial," "anti-fungal" and grammatical equivalent terms refer to a composition of the present invention which is capable of inhibiting the growth of a microorganism or kill a microorganism. Anti-microbial compositions can have microbial-static effects and/or microbial-cidal effects.

The anti-microbial composition of the present invention comprises or consists essentially of a quaternized chitosan derivative having a polymerizable organic moiety and an amphoteric surfactant, which composition is capable of decreasing the number of live microbes on the surface of the skin of a mammal.

The term "microbes" or "microbial" as used herein refers to species of bacteria, fungi, viruses and protozoa that can be treatable with the anti-microbial compositions of the present invention. For example, the anti-microbial compositions of the present invention are useful against bacterial cells such as gram positive bacteria including, but not limited to, a *Bacillus* species, e.g., *Bacillus alkalophilus*, *Bacillus amyloliquefaciens, Bacillus brevis, Bacillus circulans, Bacillus clausii*, *Bacillus coagulans, Bacillus lautus, Bacillus lentus, Bacillus licheniformis, Bacillus megaterium*, *Bacillus stearothermophilus, Bacillus subtilis,* and *Bacillus thuringiensis*; or a *Corynebacterium* species; or a *Streptomyces* species, e.g., *Streptomyces lividans, Streptomyces murinus*; or a *Streptococcus* species eg *Streptococcus pyogenes, Streptococcal intertrigo, Streptococcus iniae,* or *Streptococcus pneumoniae;* or a *Staphylococcus* species such as *Staphylococcus aureus, Staphlococcus intermedius, Staphylococcus epidermidis*; or a *Enterococcus* species such as *Enterococcus faecium*; or gram negative bacteria such as a *Salmonella* species such as *Salmonella typhi*; or a *Escherichia species* such as *Escherichia coli*; or a *Vibrio* species such as *Vibrio cholerae*; or a *Neisseria* species, such as *Neisseria meningitidis* and *Neisseria gonorrhoea*; or a *Pseudomonas* species such as *Pseudomonas aeruginosa.*

The concentration of the quaternized chitosan derivative used in the composition of the present inventions will depend upon many factors as discussed herein. However, as discussed *supra* a typically range is from about 2% w/v to about 4% w/v. This amount can vary depending on the specific chitosan derivative and/or amphoteric surfactant used in the composition, the form of the composition used (e.g., shampoo, cream, lotion and the like), the microbial species targeted, and other parameters that would be apparent to one of skill in the art. One of skill in the art would readily be able to determine the amount for a given application based on the general knowledge in the art and guidance provided in the procedures in the Examples given below.

Concentrations of the quaternized chitosan derivative used are of about, for example, 2, 20, 30, 40, 50, 60, 70, 80, 90, 100, 110, 125, 130, 140, 150, 175, 200, 225, 250, 300, 350, 400, 450, 500, 600, 750, 800, 1000, 1100, 1250, 1425, 1500, 1750, 2000, 3000, 3500, 4000, 4500, 5000, 6000, 7000, 8000, 9000, 10,000, 11,000 12,000, 13,000 15,000, 20,000, 30,000, 35,000, 40,000 or 50,000 ppm can be used in the compositions and methods of the present invention.

In one embodiment, the composition of the invention is a pharmaceutical composition. As used herein, a "pharmaceutical composition" refers to a composition that is employed to prevent, reduce in intensity, cure or otherwise treat a target microbial infection of the epithelia of a mammal.

The term "epithelia" or "epithelial" or "epithelial tissues" as used throughout the specification and claims is meant to include skin, mucosal membranes, hair and/or fur of a mammal. Thus, the present invention offers compositions useful for treating or preventing a microbial infection of the skin or a mucosal membrane.

In another embodiment, the composition of the invention is a cosmetic composition. As used herein a "cosmetic composition" refers to a composition that is intended to be rubbed, poured, sprinkled, or sprayed on, introduced into, or otherwise applied to a mammal or any part thereof for cleansing, beautifying, promoting attractiveness, or altering the appearance.

In another embodiment, the composition of the invention is a cosmeceutical composition. As used herein the term "cosmeceutical composition" refers to a composition that is employed as both a cosmetic composition and as a pharmaceutical composition.

In another aspect of the present invention, the composition of the present invention includes a carrier. As used herein "carrier" describes a material that does not abrogate the biological activity and properties of the composition of the present invention. Carriers must be of sufficiently high purity and of sufficiently low toxicity to render them suitable for administration to the mammal being treated. The carrier can be inert, or it can possess pharmaceutical benefits, cosmetic benefits or both.

Some non-limiting representative examples of carriers include moisturizing agents or humectants, pH adjusting agents, a deodorant agent, fragrances, hair conditioning agents, chelating agents, preservatives, emulsifiers, thickeners, solubilizing agents, buffering agents, chelating agents, antioxidants, stabilizers, penetration enhancers, anti-irritants, colourants and additional surfactants other than the amphoteric surfactant of the present invention.

As used herein a "moisturizing agent" is a substance that adds or restores moisture to the skin. Representative examples of moisturizing or humectant agents that are usable in the present invention include, without limitation, guanidine, glycolic acid and glycolate salts (e.g. ammonium salt and quaternary alkyl ammonium salt), aloe vera in any of its variety of forms (e.g., aloe vera gel), allantoin, urazole, polyhydroxy alcohols such as sorbitol, glycerol, hexanetriol, propylene glycol, butylene glycol, hexylene glycol and the like, polyethylene glycols, sugars and starches, sugar and starch derivatives (e.g., alkoxylated glucose), hyaluronic acid, lactamide monoethanolamine, acetamide monoethanolamine and any combination thereof.

As is widely recognized in the art, since the pH of the skin is 5.5, compositions for topical skin application (to avoid irritation) should preferably have a pH value of between 4.0 and 7.0, preferably between 5.0 and 6.0, most preferably about 5.5 or substantially 5.5. Hence, a pH adjusting composition is typically added to bring the pH of the composition to the desired value. The compositions of the present invention therefore preferably are formulated to have a pH value that ranges between about 4.0 and about 7.0, more preferably between about 5.0 and about 6.0.

Suitable pH adjusting agents include, for example, but are not limited to, one or more adipic acids, glycines, citric acids, calcium hydroxides, magnesium aluminometasilicates, buffers or any combinations thereof.

As used herein "deodorant agent" refers to a substance for inhibiting or masking perspiration or other bodily odours. Representative examples of deodorant agents that are usable in the context of the present invention include, without limitation, quaternary ammonium compounds such as cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmlthyl sarcosine, lauroyl sarcosine, N-myristoyl glycine, potassium N-lauryl sarcosine, stearyl, trimethyl ammonium chloride, sodium aluminum chlorohydroxy lactate, tricetylmethyl ammonium chloride, 2,4,4'-trichloro-2'-hydroxy diphenyl ether, diaminoalkyl amides such as L-lysine hexadecyl amide, heavy metal salts of citrate, salicylate, and piroctose, especially zinc salts, and acids thereof, heavy metal salts of pyrithione, especially zinc pyrithione and zinc phenolsulfate. Other deodorant agents include, without limitation, odour absorbing materials such as carbonate and bicarbonate salts, e.g. as the alkali metal carbonates and bicarbonates, ammonium and tetraalkylammonium carbonates and bicarbonates, especially the sodium and potassium salts, or any combination of the above. Antiperspirant agents can be incorporated in the compositions of the present invention either in a solubilized or a particulate form and include, for example, aluminium or zirconium astringent salts or complexes.

As used herein "fragrance" refers to a substance having a pleasant aroma. Suitable fragrances include, but are not limited to, eucalyptus oil, camphor synthetic, peppermint oil, clove oil, lavender, chamomile and the like.

Suitable hair conditioning agents that can be used in the context of the present invention include, for example, one or more collagens, cationic surfactants, modified silicones, proteins, keratins, dimethicone polyols, quaternary ammonium compounds, halogenated quaternary ammonium compounds, alkoxylated carboxylic acids, alkoxylated alcohols, alkoxylated amides, sorbitan derivatives, esters, polymeric ethers, glyceryl esters, or any combinations thereof.

Chelating agents are optionally added to the compositions of the present invention so as to enhance the preservative or preservative system. Preferred chelating agents are mild agents, such as, for example, ethylenediaminetetraacetic acid (EDTA), EDTA derivatives, or any combination thereof.

Suitable preservatives for use in the compositions of the present composition include, without limitation, one or more alkanols, disodium EDTA (ethylenediamine tetraacetate), EDTA salts, EDTA fatty acid conjugates, isothiazolinone, parabens such as methylparaben and propylparaben, propylene glycols, sorbates, urea derivatives such as diazolindinyl urea, or any combinations thereof.

"Emulsifiers" as used herein promote the formation and stabilization of an emulsion. Suitable emulsifiers may be natural materials, finely divided solids, or synthetic materials. Natural emulsifying agents may be derived from either animal or vegetable sources. Those from animal sources include gelatin, egg yolk, casein, wool fat, or cholesterol. Those from vegetable sources include acacia, tragacanth, chondrus, or pectin. Vegetable sources specifically from cellulose derivatives include methyl cellulose and carboxymethyl cellulose to increase the viscosity. Finely divided emulsifiers include bentonite, magnesium hydroxide, aluminium hydroxide, or magnesium trisylicate. Synthetic agents include anionic, cationic or non-ionic agents. Particularly useful are sodium lauryl sulfate, benzalkonium chloride or polyethylene glycol 400 monostearate, or any combinations thereof.

"Thickeners" as used herein refer to agents that make the composition of the present invention dense or viscous in consistency. Suitable thickeners that can be used in the context of the present invention include, for example, non-ionic water-soluble polymers such as hydroxyethylcellulose (commercially available under the Trademark Natrosol™ 250 or 350), cationic water-soluble polymers such as Polyquat 37 (commercially available under the Trademark Synthalen™), fatty alcohols, fatty acids, anionic polymers, and their alkali salts and mixtures thereof.

As used herein "solubilizing agents" are those substances that enable solutes to dissolve. Representative examples of solubilizing agents that are usable in the context of the present invention include, without limitation, complex-forming solubilizers such as citric acid, ethylenediamine-tetraacetate, sodium meta-phosphate, succinic acid, urea, cyclodextrin, polyvinylpyrrolidone, diethylammonium-ortho-benzoate, and micelle-forming solubilizers such as TWEEN™ and spans, e.g., TWEEN 80™. Other solubilizers that are usable for the compositions of the present invention are, for example, polyoxyethylene sorbitan fatty acid ester, polyoxyethylene n-alkyl ethers, n-alkyl amine n-oxides, polyoxamers, organic solvents, such as acetone, phospholipids and cyclodextrin.

A "penetration enhancer" is an agent known to accelerate the delivery of a substance through the skin. Suitable penetration enhancers usable in the present invention include, but are not limited to, dimethylsulfoxide (DMSO), dimethyl formamide (DMF), allantoin, urazole, N,N-dimethylacetamide (DMA), decylmethylsulfoxide (C₁₀MSO), polyethylene glycol monolaurate (PEGML), propylene glycol (PG), propylene glycol monolaurate (PGML), glycerol monolaurate (GML), lecithin, the 1-substituted azacycloheptan-2-ones, particularly 1-n-dodecylcyclazacycloheptan-2-one (available under the trademark Azone™ from Whitby Research Incorporated, Richmond, Va.), alcohols, and the like. The permeation enhancer may also be a vegetable oil. Such oils include, for example, safflower oil, cottonseed oil and corn oil.

Additional thickeners, penetration enhancers and other adjuvants may generally be found in Remington's Pharmaceutical Sciences, 18th or 19th editions, published by the Mack Publishing Company of Easton, PA.

As used herein, an "anti-irritant" is an agent that prevents or reduces soreness, roughness, or inflammation of a bodily part. Suitable anti-irritants that can be used in the context of the present invention include, for example, steroidal and non-steroidal anti-inflammatory agents or other materials such as aloe vera, chamomile, alpha-bisabolol, cola nitida extract, green tea extract, tea tree oil, licoric extract, allantoin, caffeine or other xanthines, glycyrrhizic acid and its derivatives.

The presently known anti-irritants can be divided into water-soluble anti-irritants and water-insoluble anti-irritants. Representative examples of such compositions are described, for example, in U.S. Pat. No. 5,482,710.

Colourants may also be used in the compositions of the invention. Colourants include pigments or dyes or a combination thereof as the cosmetic benefit requires. Preferred pigments include, but are not limited to, iron oxides, and titanium oxides. Suitable dyes include FD&C approved colourants, D&C approved colourants, and those approved for use in Europe and Japan. See Marmion, D. M., Handbook of US Colorants for Food, Drugs, CosmeticsCosmetics, and Medical Devices, 3rd ed, 1991 .

If buffering agents are required then compounds that can maintain a desired pH in an aqueous environment are used. Examples include, but are not limited to, boric acid, citric acid, lactic acid, fumaric acid, phosphoric acid, and salts thereof. See also CFTA Dictionary 1733-1734.

In some embodiments, it may be desirable to use "chelating agents", which are compounds that can complex and subsequently inactivate ions in the anti-microbial composition of the present invention and the chelating agents are used in an amount which produces the desired function provided that the amount does not affect the stability of the quaternized chitosan derivatives and amphoteric surfactants. Examples include citric acid, disodium EDTA, pentapotassium triphosphate, and phytic acid. See also CFTA Dictionary 1734-1735.

In order to prevent the degradation caused by oxidation, antioxidants may be included in the antimicrobial agents and the antioxidants are used in an amount which produces the desired function provided that the amount does not affect the stability of the quaternized chitosan derivatives and amphoteric surfactants. Antioxidants include, but are not limited to free radical scavengers and reducing agents such as, acetyl cysteine, ascorbic acid, butylated hydroxytoluene, green tea extract, caffeic acid, cysteine, tocopherol, ubiquinone, and propyl gallate, preferably butylated hydroxytoluene ("BHT"). See CFTA Dictionary 1727.

Stabilizers may also include, but are not limited to, C₁₀-₃₀ Alkyl PEG-20 itaconate copolymer, long chain acyl derivatives (including, but not limited, to ethylene glycol distearate and ethylene glycol monostearate), esters of long chain fatty acids (including but not limited to stearyl stearate), alkyl dimethylamine oxides, methylcellulose, hydroxybutyl methylcellulose, hydroxypropylcellulose, hydroxypropyl methylcellulose, hydroxyethyl cellulose, distearyl phthalic amide (e.g. Stephan SAB-2), di(hydrogenated) tallow phthalic amide, primary amines with a fatty alkyl moiety of at least 16 carbons (including but not limited to palmitate amine or stearamine), polyacrylic acids, polysaccharide gums (including but not limited to Xanthan Gum), colloidal clays (including but not limited to benzyl dimethyl hydrogenated tallow ammonium montmorillonite), colloidal silica, triethanolamne, ethanol, cetyl alcohol, cetrimonium bromide, citric acid, cyclomethicone, dimethicone, ceteth 20, ceteareth 20, caprylic/capric triglycerides, PEG 40 polyhydroxystearate, polyvinyl pyrrolidone, acetum, glyceryl stearate, xanthan gum, geranium oil, lavender oil, eucalyptus oil, tea tree oil, lemon oil, anise oil, DEA cetyl phosphate, wool alcohols, octyl stearate, carnauba wax; ozokerite, carbomer, phenoxyethanol, methyl parabens and propyl parabens and mixtures thereof. While the amount of stabilizer used can be readily determined by those skilled in the art, suitable ranges include between about 1% to about 10% v/v.

"Additional surfactants" as used herein are surface-active substances, such as a detergent: Suitable additional surfactants for use with the inventive compositions include, but are not limited to, sarcosinates, glutamates, sodium alkyl sulfates, ammonium alkyl sulfates, sodium alkyleth sulfates, ammonium alkyleth sulfates, ammonium laureth-n-sulfates, sodium laureth-n-sulfates, isothionates, glycerylether sulfonates, sulfosuccinates and combinations thereof. More preferably, the additional surfactant is selected from the group consisting of sodium lauroyl sarcosinate, monosodium lauroyl glutamate, sodium alkyl sulfates, ammonium alkyl sulfates, sodium alkyleth sulfates, ammonium alkyleth sulfates, and combinations thereof.

In a preferred embodiment, a pharmaceutically acceptable carrier is included in the composition. As used herein "a pharmaceutically acceptable carrier" is any substantially non-toxic carrier conventionally useable for topical administration of pharmaceuticals in which the quaternized chitosan derivatives and amphoteric surfactants will remain stable and bioavailable when applied directly to skin or mucosal surfaces.

In another, preferred, embodiment, the compositions of the present invention include a cosmetically acceptable carrier. As used herein the phrase "cosmetically acceptable carrier" refers to a substantially non-toxic carrier, conventionally useable for the topical administration of cosmetics, with which the quaternized chitosan derivatives and amphoteric surfactants will remain stable and bioavailable. It will be understood that cosmetically acceptable carriers and pharmaceutically acceptable carriers are similar, if not often identical, in nature.

Suitable pharmaceutically acceptable carriers include water, petroleum jelly (Vaseline™), petroleum, mineral oil, vegetable oil, animal oil, organic and inorganic waxes, such as microcrystalline, paraffin and ozocerite wax, natural polymers, such as xanthanes, gelatin, cellulose, collagen, starch, or gum arabic, alcohols, polyols, and the like. Also included are the carriers described hereinabove.

Suitable cosmetically acceptable carriers are described in the CTFA International Cosmetic Ingredient Dictionary and Handbook, 8th edition, edited by Wenninger and Canterbery, (The Cosmetic, Toiletry, and Fragrance Association, Inc., Washington, D.C., 2000), which is herein incorporated by reference. Also included are the carriers described hereinabove.

In another embodiment, the compositions of the present invention can further include one or more additional compatible active ingredients which are aimed at providing the composition with another pharmaceutical, cosmeceutical or cosmetic effect, in addition to that provided by the quaternized chitosan derivatives and amphoteric surfactants of the inventive composition. "Compatible" as used herein means that the components of such a composition are capable of being combined with each other in a manner such that there is no interaction that would substantially reduce the efficacy of the composition under ordinary use conditions.

As used herein, the phrase "additional active ingredient" refers to an agent, other than the quaternized chitosan derivative compound and/or amphoteric surfactant of the inventive composition, that exerts a pharmacological, dermatological or any other beneficial activity. It is to be understood that "other beneficial activity" may be one that is only perceived as such by the subject using the inventive compositions.

Compositions according to the present invention, which further include one or more additional active ingredients, can therefore be further efficiently used, in addition to their use as a treatment for an epithelial-related bacterial infection or in the treatment of any medical, cosmetic and/or cosmeceutical condition in which applying the additional active ingredient is beneficial.

Preferred additional active ingredients according to the present invention include, without limitation, one or more, in any combination, of a protective agent, an emollient, an astringent, an irritant, a keratolytic, a sun screening agent, an antibiotic agent, an antifungal agent, an antiviral agent, an antiprotozoal agent, an anaesthetic agent, a steroidal anti-inflammatory agent, a non-steroidal anti-inflammatory agent, an anti-pruritic agent, an anti-oxidant agent, a chemotherapeutic agent, an anti-histamine agent, and a cleansing agent.

In the broadest pharmacological sense, a "protective" is any agent that isolates the exposed surface of the skin or other membrane from harmful or annoying stimuli. Protectives as described herein may take the form of dusting powders, adsorbents, mechanical protective agents, and plasters. Dusting powders are relatively inert and insoluble materials that are used to cover and protect epithelial surfaces, ulcers and wounds. Usually, these substances are finely subdivided powders that absorb moisture and can act as a desiccant. The absorption of skin moisture decreases friction and also discourages certain bacterial growth. Some of the materials used as protective adsorbents include bentonite, insoluble salts of bismuth, boric acid, calcium carbonate, (precipitated), cellulose, corn starch, magnesium stearate, talc, titanium dioxide, zinc oxide, and zinc stearate.

Protectives also can be administered to the skin to form an adherent, continuous film that may be flexible or semi-rigid depending on the materials and the formulations as well as the manner in which they are applied. This material may serve several purposes including providing occlusion from the external environment, providing chemical support, and serving as vehicles for other medicaments. Mechanical protectives are generally either collodions or plasters. Examples include aluminium hydroxide gel, collodium, dimethicone, petrolatum gauze, absorbable gelatin film, absorbable gelatin sponge, zinc gelatin, kaolin, lanolin, anhydrous lanolin, mineral oil, mineral oil emulsion, mineral oil light, olive oil, peanut oil, petrolatum, silicones, hydrocolloids and the like.

Preferably, protectives included in the composition of the invention are demulcents. Demulcents are protective agents employed primarily to alleviate irritation, particularly mucous membranes or abraded tissues. They often are applied to the surface in a viscid, sticky preparation that covers the area readily and may be medicated. A number of chemical substances possess demulcent properties. These substances include the alginates, mucilages, gums, dextrins, starches, certain sugars, and polymeric polyhydric glycols. Others include acacia, agar, benzoin, carbomer, gelatin, glycerin, hydroxyethyl cellulose, hydroxypropyl cellulose, hydroxypropyl methylcellulose, propylene glycol, sodium alginate, tragacanth, and the like.

"Emollients" are generally bland, fatty or oleaginous materials which can be applied locally, particularly to the skin. Emollients increase the tissue moisture content, thereby rendering the skin softer and more pliable. Increased moisture content in the skin can be achieved by preventing water loss with an occlusive water-immiscible barrier, by increasing the water-holding capacity in the skin with humectants, or by altering the desquamation of the outermost skin layer, the stratum corneum. Useful emollients include lanolin, spermaceti, mineral oil, paraffin, petrolatum, white ointment, white petroleum, yellow ointment. Also included are vegetable oils, waxes, cetyl alcohol, glycerin, hydrophilic petrolatum, isopropyl myristate, myristyl alcohol, and oleyl alcohol.

"Astringents" are locally applied, generally protein precipitants, that have such a low cell penetrability that the action essentially is limited to the cell surface and interstitial spaces. The astringent action is accompanied by contraction and wrinkling of the tissue and by blanching. Astringents are used therapeutically to arrest haemorrhage by coagulating the blood, to promote healing, to toughen the skin or to decrease sweating. The principal components of astringents are salts of aluminium, zinc, manganese, iron or bismuth.

An "irritant" is a material that acts locally on the skin to induce, based on irritant concentration, hyperemia, inflammation, and desiccation. Irritant agents include, but are not limited to, alcohol, aromatic ammonia spirits, benzoin tincture, camphor capsicum, and coal tar extracts. Preferably, the irritant is a rubefacient. As used herein "rubefacients" are agents that induce hyperemia, wherein hyperemia means an increased amount of blood in a body part or organ. Rubefaction, which is induced by rubefacients, results from increased circulation to an injured area and is accompanied by a feeling of comfort, warmth, itching and hyperesthesia.

"Keratolytics" (desquamating agents) act to remove outer layers of the stratum corneum. This is particularly useful in hyperkeratotic areas. The keratolytics include benzoyl peroxide, fluorouracil, resorcinol, salicylic acid, tretinoin, and the like.

Representative examples of sun screening agents usable in context of the present invention include, without limitation, p-aminobenzoic acid and its salts and derivatives thereof (ethyl, isobutyl, glyceryl esters; p-dimethylaminobenzoic acid); anthranilates (i.e., o-amino-benzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-propylene glycol esters); cinnamic acid derivatives (menthyl and benzyl esters, a-phenyl cinnamonitrile; butyl cinnamoyl pyruvate); dihydroxycinnamic acid derivatives (umbelliferone, methylumbelliferone, methylaceto-umbelliferone); trihydroxy-cinnamic acid derivatives (esculetin, methylesculetin, daphnetin, and the glucosides, esculin and daphnin); hydrocarbons (diphenylbutadiene, stilbene); dibenzylacetone and benzylacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl)ether; hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene boman-2-one) and 4-isopropyl-di-benzoylmethane, and any combination thereof.

The term "antibiotic agent" as used herein means any of a group of chemical substances having the capacity to inhibit the growth of, or to destroy bacteria, and other microorganisms, used chiefly in the treatment of infectious diseases. Examples of antibiotic agents include, but are not limited to, Penicillin G; Methicillin; Nafcillin; Oxacillin; Cloxacillin; Dicloxacillin; Ampicillin; Amoxicillin; Ticarcillin; Carbenicillin; Mezlocillin; Azlocillin; Piperacillin; Imipenem; Aztreonam; Cephalothin; Cefaclor; Cefoxitin; Cefuroxime; Cefonicid; Cefmetazole; Cefotetan; Cefprozil; Loracarbef; Cefetamet; Cefoperazone; Cefotaxime; Ceftizoxime; Ceftriaxone; Ceftazidime; Cefepime; Cefixime; Cefpodoxime; Cefsulodin; Fleroxacin; Nalidixic acid; Norfloxacin; Ciprofloxacin; Ofloxacin; Enoxacin; Lomefloxacin; Cinoxacin; Doxycycline; Minocycline; Tetracycline; Amikacin; Gentamicin; Kanamycin; Netilmicin; Tobramycin; Streptomycin; Azithromycin; Clarithromycin; Erythromycin; Erythromycin estolate; Erythromycin ethyl succinate; Erythromycin glucoheptonate; Erythromycin lactobionate; Erythromycin stearate; Vancomycin; Teicoplanin; Chloramphenicol; Clindamycin; Trimethoprim; Sulfamethoxazole; Nitrofurantoin; Rifampin; Mupirocin; Metronidazole; Cephalexin; Roxithromycin; Co-amoxiclavuanate; combinations of Piperacillin and Tazobactam; and their various salts, acids, bases, and other derivatives. Anti-bacterial antibiotic agents include, but are not limited to, penicillins, cephalosporins, carbacephems, cephamycins, carbapenems, monobactams, aminoglycosides, glycopeptides, quinolones, tetracyclines, macrolides, and fluoroquinolones.

The term "anti-fungal agent" as used herein means any of a group of chemical substances having the capacity to inhibit the growth of or to destroy fungi. Anti-fungal agents include but are not limited to Amphotericin B, Candicidin, Dermostatin, Filipin, Fungichromin, Hachimycin, Hamycin, Lucensomycin, Mepartricin, Natamycin, Nystatin, Pecilocin, Perimycin, Azaserine, Griseofulvin, Oligomycins, Neomycin, Pyrrolnitrin, Siccanin, Tubercidin, Viridin, Butenafine, Naftifine, Terbinafine, Bifonazole, Butoconazole, Chlordantoin, Chlormidazole, Cloconazole, Clotrimazole, Econazole, Enilconazole,-Fenticonazole, Flutrimazole, Isoconazole, Ketoconazole, Lanoconazole, Miconazole, Omoconazole, Oxiconazole, Sertaconazole, Sulconazole, Tioconazole, Tolciclate, Tolindate, Tolnaftate, Fluconawle, Itraconazole, Saperconazole, Terconazole, Acrisorcin, Amorolfine, Biphenamine, Bromosalicylchloranilide, Buclosamide, Calcium Propionate, Chlorphenesin, Ciclopirox, Cloxyquin, Coparaffinate, Diamthazole, Exalamide, Flucytosine, Halethazole, Hexetidine, Loflucarban, Nifuratel, Potassium Iodide, Propionic Acid, Pyrithione, Salicylanilide, Sodium Propionate, Sulbentine, Tenonitrozole, Triacetin, Ujothion, Undecylenic Acid, and Zinc Propionate.

The term "anti-viral agent" as used herein means any of a group of chemical substances having the capacity to inhibit the replication of or to destroy viruses used chiefly in the treatment of viral diseases. Anti-viral agents include, but are not limited to, Acyclovir, Cidofovir, Cytarabine, Dideoxyadenosine, Didanosine, Edoxudine, Famciclovir, Floxuridine, Ganciclovir, Idoxuridine, Inosine Pranobex, Lamivudine, MADU, Penciclovir, Sorivudine, Stavudine, Trifluridine, Valacyclovir, Vidarabine, Zalcitabine, Zidovudine, Acemannan, Acetylleucine, Amantadine, Amidinomycin, Delavirdine, Foscamet, Indinavir, Interferon-.alpha., Interferon-.beta., Interferon-.gamma., Kethoxal, Lysozyme, Methisazone, Moroxydine, Nevirapine, Podophyllotoxin, Ribavirin, Rimantadine, Ritonavir2, Saquinavir, Stailimycin, Statolon, Tromantadine, Zidovudine (AZT) and Xenazoic Acid.

The term "anti-protozoal agent" as used herein means any of a group of chemical substances having the capacity to inhibit the growth of or to destroy protozoans used chiefly in the treatment of protozoal diseases. Examples of antiprotozoal agents, without limitation include pyrimethamine (Daraprim™) sulfadiazine, and Leucovorin.

"Anaesthetic agents" refers to agents that resulting in a reduction or loss of sensation. Non-limiting examples of anaesthetic drugs that are suitable for use in the context of the present invention include pharmaceutically acceptable salts of lidocaine, bupivacaine, chlorprocaine, dibucaine, etidocaine, mepivacaine, tetracaine, dyclonine, hexylcaine, procaine, cocaine, ketamine, pramoxine and phenol.

"Steroidal anti-inflammatory agent", as used herein, refer to any one of numerous compounds containing a 17-carbon 4-ring system and includes the sterols, various hormones (as anabolic steroids), and glycosides. Representative examples of steroidal anti-inflammatory drugs include, without limitation, corticosteroids such as hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone; diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof.

"Non-steroidal anti-inflammatory agents" refers to a large group of agents that are aspirin-like in their action, including ibuprofen (Advil)™, naproxen sodium (Aleve)™, and acetaminophen (Tylenol)™. Additional examples of non-steroidal anti-inflammatory agents that are usable in the context of the present invention include, without limitation, oxicams, such as piroxicam, isoxicam, tenoxicam, sudoxicam, and CP-14,304; disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Mixtures of these non-steroidal anti-inflammatory agents may also be employed, as well as the dermatologically acceptable salts and esters of these agents. For example, etofenamate, a flufenamic acid derivative, is particularly useful for topical application.

"Anti-pruritic agents" as used herein refers to those substances that reduce, eliminate or prevent itching. Suitable anti-pruritic agents include, without limitation, pharmaceutically acceptable salts of methdilazine and trimeprazine.

"An anti-oxidant agent" as used herein refers to a substance that inhibits oxidation or reactions promoted by oxygen or peroxides. Non-limiting examples of anti-oxidants that are usable in the context of the present invention include ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox™), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, glycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts.

"Anti-histamine agent" as used herein refers to any of various compounds that counteract histamine in the body and that are used for treating allergic reactions. Non-limiting examples of antihistamines usable in context of the present invention include chlorpheniramine, brompheniramine, dexchlorpheniramine, tripolidine, clemastine, diphenhydramine, promethazine, piperazines, piperidines, astemizole, loratadine and terfenadine.

Cleansing agents which may be use in the present invention include surfactant based cleansing agents, examples of which have been listed hereinabove. Other non-surfactant-based cleansing agents known to those of skill in the art may also be employed.

The topical compositions of the present invention can be applied locally to the skin, mucosa, hair or fur of a mammal and may be in any form including solutions, oils, creams, ointments, gels, lotions, shampoos, milks, cleansers, moisturizers, sprays and the like.

In another embodiment, the quaterized chitosan derivative and amphoteric surfactant composition, carrier and, optionally, additional active ingredients are formed into a composition comprising a solution, emulsion or gel suspension.

In some embodiments, the quaterized chitosan derivative and amphoteric surfactant composition, a pharmaceutical or cosmetic carrier and, optionally, one or more additional active ingredients are in the form of a medicated shampoo.

A medicated shampoo can be prepared by mixing a solute or dissolved substance (such as a quaterized chitosan derivative and amphoteric surfactant of the invention and, optionally, one or more active ingredient(s)) uniformly throughout a solvent carrier such as water or organic solvents, such as the alcohols (e.g. ethanol or isopropanol, acetone).

Additional compositions of the present invention using the quaterized chitosan derivatives and amphoteric surfactants can be readily prepared using technology which is known in the art such as described in Remington's Pharmaceutical Sciences, 18th or 19th editions, published by the Mack Publishing Company of Easton, PA.

According to another aspect of the present invention, there is provided a method of preparing the novel compositions described hereinabove. The process generally includes admixing the at least one quaterized chitosan derivative and at least one amphoteric surfactant, as described hereinabove, and the pharmaceutically, cosmetically or cosmeceutically acceptable carrier. In cases where additional active ingredients, as detailed above, are present in the compositions, the process includes admixing these ingredients together with the active ingredients and the carrier. The mixing technique utilized in the process of the present invention can involve any one of the known techniques for formulating topical compositions. A variety of exemplary formulation techniques that are usable in the process of the present invention is described, for example, in Harry's Cosmeticology, Seventh Edition, Edited by J B Wilkinson and R J Moore, Longmann Scientific & Technical, 1982.

According to another aspect of the present invention, there is provided a method of treating and/or preventing a medical, cosmetic and/or cosmeceutical condition associated with epithelial tissues and in particular bacterial skin infections. The method is effected by topically applying, a pharmaceutically, cosmetically or cosmeceutically effective amount of the composition of the present invention as described above onto the epithelial surface of an animal.

The term "topical" refers to administration of an inventive composition at, or immediately beneath, the point of application.

The phrase "topically applying" describes application onto one or more surfaces(s) including epithelial surfaces. "Topically applying" refers to direct application to the area of the surface to be affected. The composition may be applied by pouring, dropping, or spraying, if a liquid; rubbing on, if an ointment, lotion, cream, gel, or the like; dusting, if a powder; spraying, if a liquid or aerosol composition; or by any other appropriate means.

As used herein the terms "pharmaceutically effective amount" "cosmetically effective amount" or "cosmeceutically effective amount" refer to the amount of any of the compositions of the invention that result in a therapeutic or beneficial effect following its administration to a mammal. The pharmaceutical, cosmeceutical or cosmetic effect can be curing, minimizing, preventing or ameliorating a bacterial infection, improving the physical appearance and aesthetics (e.g., skin hydration), or may have any other pharmaceutical, cosmeceutical or cosmetic beneficial effect. The concentration of the composition of the present invention is selected so as to exert its pharmaceutical, cosmeceutical or cosmetic effect, but low enough to avoid significant side effects within the scope and sound judgment of the skilled artisan. The effective amount of the composition may vary with the particular epithelial tissue being treated, the age and physical condition of the biological subject being treated, the severity of the condition, the duration of the treatment, the nature of concurrent therapy, the specific compound, composition or other active ingredient employed, the particular carrier utilized, and like factors.

A skilled artisan can determine a pharmaceutically effective amount of the inventive compositions by determining the unit dose. As used herein, a "unit dose" refers to the amount of inventive composition required to produce a response of 50% of maximal effect (i.e. ED₅₀). The unit dose can be assessed by extrapolating from dose-response curves derived from *in vitro* or animal model test systems.

According to this aspect of the present invention, the compositions of the present invention are preferably topically applied as needed. In another preferred embodiment, the inventive compositions are topically applied between one and four times a day, more preferably twice a day (e.g., once in the morning and once in the evening). The topical application of the compositions of the present invention is preferably carried out for a time period that ranges between 1 and 30 days, more preferably for a time period of about fourteen days. Some conditions may require topical application for an indeterminate length of time.

The methods of the present invention provide for topically contacting a composition as described above with the skin, mucous membranes, hair or fur of a mammal that have been in contact with bacteria.

By "bacteria" is meant a unicellular prokaryotic microorganism that usually multiplies by cell division.

By "bacterial infection" is meant the invasion of a host mammal by bacteria. For example, the infection may include the excessive growth of bacteria that are normally present in or on the body of an animal or growth of bacteria that are not normally present in or on the animal. More generally, a bacterial infection can be any situation in which the presence of a bacterial population(s) is damaging to a host mammal. Thus, a mammal is "suffering" from a bacterial infection when an excessive amount of a bacterial population is present in or on the mammal's body, or when the presence of a bacterial population(s) is damaging the cells or other tissue of the mammal.

The following examples, which describe exemplary techniques and experimental results, are provided for the purpose of illustrating the invention, and should not be construed as limiting.

### EXAMPLE 1 MIC₉₀ DETERMINATION OF ANTI-MICROBIAL COMPOSITION

1% w/v (∼10,000 ppm) of quaternized chitosan derivative (DMDC-Q-g-EM) was mixed with disodium cocamphodiacetate (DCC) (between 5% and 25% in aqueous solution) to produce a trial anti-microbial composition which was then validated by means of MIC₉₀ determination.

Briefly, bacterial cells were inoculated in culture medium (MH broth) for overnight growth. The suspension of bacteria was spread on LB agar plate to form discrete colonies. Purified bacteria were then cultured from a single colony by inoculation in to MH broth and grown overnight at 37°C to achieve mid-log phase and then diluted to 10⁴ to 10⁵ CFU ml⁻¹.

The anti-microbial composition was then dissolved in MH broth at a desired concentration. A two-fold dilution series of 100µl product solution in MH broth was made on 96-well microplate from no. 1 to 10. Wells no. 11 and 12 were used as negative/positive controls. Positive control was without product and negative control was without bacterial inoculums. Bacteria suspension was diluted to 10⁴-10⁵ CFU ml⁻¹. 100µl of bacterial suspension was added into wells no. 1 to 10 and the positive control well, while 100µl sterile MH broth was added in the negative control. The plates were incubated at 37°C for 24h or other desired time.

The growth of bacteria in the microplate was observed by microplate spectrometer or visually. The absorbance at 600nm was measured with a microplate spectrometer (BIO-RAD, US). MICs were determined as the lowest concentration that inhibited cell growth by more than 90%.

Table 1 shows the effect of the anti-microbial composition on *S. aureus ATCC6538 and E. coli* ATCC8939. It can be seen that there was a significant improvement in the MIC₉₀ values when 4% w/v of quaternized chitosan derivative was combined with disodium cocamphodiacetate (test anti-microbial composition).

**TABLE 1: SUMMARY OF MIC₉₀ VALUES**

| **Bacterial strains** | **MIC₉₀ values in µg per ml** | |
|---|---|---|
| | Test anti-microbial composition | 4% w/v of quaternized chitosan derivative Alone |
| *S. aureus ATCC6538* | 40 | 200 |
| *E. coli* ATCC8939 | 20 | 200 |

The combination of 4% w/v of quaternized chitosan derivative and disodium cocamphodiacetate (test anti-microbial composition) was tested against wild strains of the causal agent of canine pyoderma, namely *Staphylococcus pseudintermedius* (80% of cases) and *Staphylococcus aureus* (15% of cases). The further improvement observed in the MIC₉₀ values for the more important *S. pseudintermedius* strains demonstrated a synergistic effect of adding disodium cocamphodiacetate to 4% w/v of quaternized chitosan derivative.

**TABLE 2: SUMMARY OF MIC₉₀ VALUES**

| **Causal agents of *C*anine Pyoderma** | **MIC₉₀ values in µg per ml** |
|---|---|
| *Staphylococcus pseudintermedius AB7* | 20 |
| *Staphylococcus pseudintermedius AC19* | 20 |
| *Staphylococcus pseudintermedius AC3* | 20 |
| *Staphylococcus aureus SCKA1* | 40 |
| *Staphylococcus aureus SCKA2* | 40 |
| *Staphylococcus aureus AT29* | 40 |

The combination 4% w/v of quaternized chitosan derivative with disodium cocamphodiacetate (test anti-microbial composition) was further tested on the micro-flora (principally *S. Pseudintermedius*) of the skin of 6 dogs suffering from canine pyoderma. Results obtained demonstrated a decrease in the micro-flora in all the 6 dogs by more than 50 times on the average:

**TABLE 3: SUMMARY OF BACTERIAL LOAD ON THE SKIN (IN LOG₁₀₋)**

| Days post-treatment | Dog 1 | Dog 2 | Dog 3 | Dog 4 | Dog 5 | Dog 6 |
|---|---|---|---|---|---|---|
| Day 0 | 4.42 | 4.37 | 2.84 | 3.25 | 4.13 | 4.18 |
| Day 8 | 3.69 | 3.88 | 2.08 | 2.00 | 2.04 | 3.94 |

### EXAMPLE 2 ACUTE ORAL TOXICITY IN RATS

The procedure adopted was from the OECD Guideline for Testing of Chemicals, Acute Oral Toxicity - Acute Toxic Class Method 423.

For each group of animals tested with an aqueous solution of the test anti-microbial composition these procedures were followed:
1. Three healthy, female nulliparous and non-pregnant Wistar (WI) rats, 6-7 weeks old, were obtained and acclimatized at the national institute of laboratory-animal-based toxicity studies in Singapore, for a minimum of 5 days. All three animals were caged together in an Individually Ventilated Cage (IVC). Room temperature was kept at 22-26°C, humidity at 40-70% with 12 hour light and 12 hour dark cycle. They were fed a standard laboratory diet for rodents which had been irradiated. Drinking water was offered *ad labitum* through water bottles that had been autoclaved. Before test substance was orally administered, feed (but not water) was withheld overnight. Body weights were recorded just before dosing.
2. Each group of 3 female rats was dosed as a single oral gavage with the test anti-microbial composition to give dose equivalents of 50mg/kg, 150mg/kg and 300mg/kg per rat. Volumes administered did not exceed 1ml/100g body weight per rate and maintained as constant as possible.
3. After substance administration, food (but not water) was withheld for a further 3-4 hours.
4. All animals were observed at last once during the first 30 minutes after dosing, then periodically during the first 24 hours, with special attention given during the first 4 hours. Thereafter, they were observed daily for a total of 14 days, except where they need to be removed from the study and humanely killed for animal welfare reasons or were found dead.
5. Individual body weights were determined shortly before substance administration, and at least weekly thereafter. All surviving animals were weighed, humanely euthanized and necropsied at the end of the study.

### Results:

**1. Mortality:** No mortalities were observed in groups, including a subsequent group of 3 rats dosed at 300mg/kg.
**2. Physical observations:** All rats appeared normal thereafter till euthanized. No untoward clinical signs were observed during this period.
**3. Necropsy:** Euthanasia was carried out by overdoes of CO inhalation. All rats did not have significant lesions observed on gross pathology.

**Conclusion:** The LD₅₀ for the test anti-microbial composition in rats cannot be determined accurately from this method but is considered to be greater than 300mg/kg body weight.

## Claims

1. A topical anti-microbial composition comprising a quaternized chitosan derivative having a polymerizable organic moiety and an amphoteric surfactant, the amphoteric surfactant being any one of the following surfactants: N- alkylglycines, N-alkylaminopropionic acids, N-alkylaminobutyric acids, N-alkyliminodipropionic acids, N-hydroxyethyl-N -alkylarnidopropylglycines, N- alkyltaurines, N-alkylsarcosines, 2-alkylaminopropionic acids, each having about 8 to 24 C atoms in the alkyl group, alkylaminoacetic acids each having about 8 to 24 C atoms in the alkyl group, N - cocoalkyl aminopropionate, cocoacyl aminoethyl aminopropionate, C₁₂-C₁₈ acyl sarcosine, N-alkyl-N,N-dimethylammonium glycinates, for example, cocoalkyl dimethylammonium glycinate, N-acyl-aminopropyl-N,N- dimethylammoniuro glycinates, for example, cocoacyl aminopropyl, dimethylammonium glycinate, 2-alkyl-3-carboxymethyl-3-hydroxyethyl imidazolines each having about 8 to 18 C atoms in the alkyl or acyl group, cocoacyl aminoethyl hydroxyethyl carboxymethyl glycinate, compounds known under the INCI name cocamidopropyl betaine, compounds known under the INCI name disodium cocoamphodiacetate.

2. A topical anti-microbial composition according to claim 1 consisting essentially of a quaternized chitosan derivative having a polymerizable organic moiety and an amphoteric surfactant.

3. A topical anti-microbial composition according to claim 1 for use as a medicament for the treatment of skin infections in mammals.

4. A topical anti-microbial composition according to claim 1 in the manufacture of a medicated·shampoo for the treatment of microbial skin infections.

5. A topical anti-microbial composition according to any one of claims 1 to 4, further comprising a carrier selected from the group consisting of a moisturizing agent, a humectant, a pH adjusting agent, a deodorant agent, a fragrance, a hair conditioning agent, a chelating agent, a preservative, a emulsifier, a thickener, a solubilizing agent, a buffering agent, a chelating agent, an antioxidant, a stabilizer, a penetration enhancer, an anti-irritant, a colourant and additional surfactant other than an amphoteric surfactant.

6. A topical anti-microbial composition according to any one of claims 1 to 5, wherein the concentration of said quaternized chitosan derivative used in said composition is between about 2% w/v and 4% w/v.

7. A topical anti-microbial composition according to any one of claims 1 to 6, further comprising an agent selected from the group consisting of a protective agent, an emollient, an astringent, an irritant, a keratolytic, a sun screening agent, an antibiotic agent, an antifungal agent, an antiviral agent, an antiprotozoal agent, an anaesthetic agent, a steroidal anti-inflammatory agent, a non-steroidal anti-inflammatory agent, an anti-pruritic agent, an anti-oxidant agent, a chemotherapeutic agent, an anti-histamine agent, and a cleansing agent.

8. A topical anti-microbial composition according to any one of claims 1 to 7, wherein the topical anti-microbial composition is effective in the treatment of a bacterial infection.

9. A topical anti-microbial composition according to claim 8, wherein the bacterial infection is caused by one or more of *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis, Bacillus thuringiensis, Corynebacterium* species, *Streptomyces lividans, Streptomyces murinus, Streptococcus pyogenes, Streptococcal intertrigo, Streptococcus iniae, Streptococcus pneumoniae, Staphylococcus aureus, Staphlococcus intermedius, Staphylococcus pseudintermedius, Staphylococcus epidermidis, Enterococcus faecium, Salmonella typhi, Escherichia coli, Vibrio cholerae, Neisseria meningitidis, Neisseria gonorrhoea* and *Pseudomonas aeruginosa.*

10. A topical anti-microbial composition according to claim 9, wherein the bacterial infection is caused by *Staphylococcus aureus* or *Staphylococcus pseudintermedius.*

11. A topical anti-microbial composition according claim 9, wherein the bacterial infection is caused by *Escherichia coli.*

12. A topical anti-microbial composition according to any one of claims 5 to 11,·wherein the chelating agent is selected from the group consisting of lactic acid, tartaric acid, adipic acid, succinic acid, citric acid, ascorbic acid, malic acid, mandelic acid, acetic acid, sorbic acid, sodium acid pyrophosphate, acidic sodium hexametaphosphate and ethylenediaminetetraacetic acid and salts thereof.

13. A topical anti-microbial composition according to any one of claims 5 to 12, wherein the stabilizer is selected from the group consisting of glyceryl monostearate, stearic acid, triethanolamne, ethanol, polysorbate 20, cetyl alcohol, stearyl alcohol, cetrimonium bromide, citric acid, cyclomethicone, dimethicone, ceteth 20, ceteareth 20, caprylic/capric triglycerides, PEG 40 polyhydroxystearate, polyvinyl pyrrolidone, acetum, glyceryl stearate, xanthan gum, geranium oil, lavender oil, eucalyptus oil, tea tree oil, lemon oil, anise oil, DEA cetyl phosphate, sodium stearate, potassium stearate, wool acohols, octyl stearate, carnauba wax, ozokerite, carbomer, phenoxyethanol, methyl parabens and propyl parabens and mixtures thereof.

14. A method of forming a topicai·anti-microbial composition comprising a quatemized chitosan derivative having polymerizable organic moieties and an amphoteric surfactant as defined in claim 1, comprising the step of mixing at least one quaternized chitosan derivative having a polymerizable organic moiety with at least one amphoteric surfactant.

15. A topical composition according to any one of claims 1 to 12 for use in a method of treating skin infections in mammals comprising the step of topically applying to said skin an effective amount of the topical composition .

16. A topical composition according to any one of claims 1 to 12 for use in a method for treating or preventing a microbial skin infection in a mammal, comprising the step of applying to said mammal the topical composition.

17. A topical composition for use according to claim 15 or 16, wherein said topical anti-microbial composition is applied to infected skin of said mammal.

## Patentansprüche

1. Topische antimikrobielle Zusammensetzung, umfassend ein quaternisiertes Chitosanderivat mit einer polymerisierbaren organischen Gruppierung und ein amphoteres Tensid, wobei es sich bei dem amphoteren Tensid um eines der folgenden Tenside handelt: N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Cocoalkylaminopropionat, Cocoacylaminoethylaminopropionat, C₁₂-C₁₈-Acylsarkosin, N-Alkyl-N,N-dimethyllammoniumlgycinate, zum Beispiel Cocoalkyldimethylammoniumglycinat, N-Acylaminopropyl-N,N-dimethylammoniumglycinate, zum Beispiel Cocoacylaminopropyldimethylammoniumglycinat, 2-Alkyl-3-carboxymethyl-3-hydroxyethylimidazoline mit jeweils etwa 8 bis 18 C-Atomen in der Alkyl- bzw. Acylgruppe, Cocoacylaminoethylhydroxyethylcarboxymethylglycinatverbindungen, die unter dem INCI-Namen Cocamidopropylbetain bekannt sind, unter dem INCI-Namen Dinatriumcocoamphodiacetat bekannte Verbindung.

2. Topische antimikrobielle Zusammensetzung nach Anspruch 1, im Wesentlichen bestehend aus einem quaternisierten Chitosanderivat mit einer polymerisierbaren organischen Gruppierung und einem amphoteren Tensid.

3. Topische antimikrobielle Zusammensetzung nach Anspruch 1 zur Verwendung als Medikament zur Behandlung von Hautinfektionen bei Säugetieren.

4. Topische antimikrobielle Zusammensetzung nach Anspruch 1 zur Herstellung eines Medizinalshampoos zur Behandlung mikrobieller Hautinfektionen.

5. Topische antimikrobielle Zusammensetzung nach einem der Ansprüche 1 bis 4, weiterhin umfassend einen Träger ausgewählt aus der Gruppe bestehend aus einem Befeuchtungsmittel, einem Feuchthaltemittel, einem Mittel zum Einstellen des pH-Wertes, einem deodorierenden Mittel, einem Duftstoff, einem Haarpflegemittel, einem Chelator, einem Konservierungsstoff, einem Emulgator, einem Verdickungsmittel, einem Solobilisator, einem Puffer, einem Chelator, einem Antioxidationsmittel, einem Stabilisator, einem Penetrationsverbesserer, einem reizlindernden Mittel, einem Farbstoff und einem zusätzlichen Tensid, bei dem es sich nicht um ein amphoteres Tensid handelt.

6. Topische antimikrobielle Zusammensetzung nach einem der Ansprüche 1 bis 5, wobei die Konzentration des in der Zusammensetzung eingesetzten quaternisierten Chitosanderivats zwischen etwa 2% (w/v) und 4% (w/v) liegt.

7. Topische antimikrobielle Zusammensetzung nach einem der Ansprüche 1 bis 6, weiterhin umfassend ein Mittel ausgewählt aus der Gruppe bestehend aus einem schützenden Mittel, einem Emolliens, einem Astringens, einem Reizstoff, einem Keratolytikum, einem Sonnenschutzmittel, einem Antibiotikum, einem Antipilzmittel, einem antiviralen Mittel, einem Mittel gegen Protozoen, einem Anästhetikum, einem steroidalen entzündungshemmenden Mittel, einem nichtsteroidalen entzündungshemmenden Mittel, einem juckreizstillenden Mittel, einem Antioxidationsmittel, einem Chemotherapeutikum, einem Antihistaminikum und einem Reinigungsmittel.

8. Topische antimikrobielle Zusammensetzung nach einem der Ansprüche 1 bis 7, wobei die topische antimikrobielle Zusammensetzung bei der Behandlung einer bakteriellen Infektion wirksam ist.

9. Topische antimikrobielle Zusammensetzung nach Anspruch 8, wobei die bakterielle Infektion durch einen oder mehrere von *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis, Bacillus thuringiensis, Corynebacterium-*Arten, *Streptomyces lividans, Streptomyces murinus, Streptococcus pyogenes, Streptococcal intertrigo, Streptococcus iniae, Streptococcus pneumoniae, Staphylococcus aureus, Staphlococcus intermedius, Staphylococcus pseudintermedius, Staphylococcus epidermidis, Enterococcus faecium, Salmonella typhi, Escherichia coli, Vibrio cholerae, Neisseria meningitidis, Neiserria gonorrhoea* und *Pseudomonas aeruginosa* verursacht wird.

10. Topische antimikrobielle Zusammensetzung nach Anspruch 9, wobei die bakterielle Infektion durch *Staphylococcus aureus* oder *Staphylococcus pseudintermedius* verursacht wird.

11. Topische antimikrobielle Zusammensetzung nach Anspruch 9, wobei die bakterielle Infektion durch *Escherichia coli* verursacht wird.

12. Topische antimikrobielle Zusammensetzung nach einem der Ansprüche 5 bis 11, wobei der Chelator aus der aus Milchsäure, Weinsäure, Adipinsäure, Bernsteinsäure, Citronensäure, Ascorbinsäure, Äpfelsäure, Mandelsäure, Essigsäure, Sorbinsäure, Dinatriumdihydrogendiphosphat, saurem Natriumhexametaphosphat und Ethylendiamintetraessigsäure und Salze davon bestehenden Gruppe ausgewählt ist.

13. Topische antimikrobielle Zusammensetzung nach einem der Ansprüche 5 bis 12, wobei der Stabilisator aus der aus Glycerylmonostearat, Stearinsäure, Triethanolamin, Ethanol, Polysorbat 20, Cetylalkohol, Stearylalkohol, Cetrimoniumbromid, Citronensäure, Cyclomethicon, Dimethicon, Ceteth 20, Ceteareth 20, Caprylsäure-/Caprinsäuretriglyceriden, PEG 40 Polyhdroxystearat, Polyvinylpyrrolidon, Acetum, Glycerylstearat, Xanthan, Geraniumöl, Lavendelöl, Eukalyptusöl, Teebaumöl, Zitronenöl, Anisöl, DEA-Cetylphosphat, Natriumstearat, Kaliumstearat, Wollalkoholen, Octylstearat, Carnaubawachs, Ozokerit, Carbomer, Phenoxyethanol, Methylparabenen und Propylparabenen und Mischungen davon bestehenden Gruppe ausgewählt ist.

14. Verfahren zur Bildung einer ein quaternisiertes Chitosanderivat mit polymerisierbaren organischen Gruppierungen und ein amphoteres Tensid umfassenden, wie in Anspruch 1 definierten topischen antimikrobiellen Zusammensetzung, umfassend den Schritt des Mischens mindestens eines quaternisierten Chitosanderivats mit einer polymerisierbaren organischen Gruppierung mit mindestens einem amphoteren Tensid.

15. Topische Zusammensetzung nach einem der Ansprüche 1 bis 12 zur Verwendung in einem Verfahren zur Behandlung von Hautinfektionen bei Säugetieren, umfassend den Schritt der topischen Anwendung einer wirksamen Menge der topischen Zusammensetzung auf der Haut.

16. Topische Zusammensetzung nach einem der Ansprüche 1 bis 12 zur Verwendung in einem Verfahren zur Behandlung oder Prävention einer mikrobiellen Hautinfektion bei einem Säugetier, umfassend den Schritt der Anwendung der topischen Zusammensetzung bei dem Säugetier.

17. Topische Zusammensetzung zur Verwendung nach Anspruch 15 oder 16, wobei die topische antimikrobielle Zusammensetzung auf infizierter Haut des Säugetiers angewendet wird.

## Revendications

1. Composition antimicrobienne topique, comprenant un dérivé de chitosane quaternisé ayant un motif organique polymérisable et un agent tensioactif amphotère, l'agent tensioactif amphotère étant l'un quelconque parmi les agents tensioactifs suivants : les N-alkylglycines, les acides N-alkylaminopropioniques, les acides N-alkylaminobutyriques, les acides N-alkylimino-dipropioniques, les N-hydroxyéthyl-N-alkylamidopropylglycines, les N-alkyltaurines, les N-alkylsarcosines, les acides 2-alkylaminopropioniques, ayant chacun d'environ 8 à 24 atomes de C dans le groupement alkyle, les acides alkylaminoacétiques ayant chacun d'environ 8 à 24 atomes de C dans le groupement alkyle, l'aminopropionate de N-cocoalkyle, l'aminopropionate de cocoacyl aminoéthyle, la C₁₂-C₁₈-acylsarcosine, les glycinates de N-alkyl-N,N-diméthylammonium, par exemple le glycinate de cocoalkyl diméthylammonium, les glycinates de N-acylaminopropyl-N,N-diméthylammonium, par exemple le glycinate de cocoacyl aminopropyl diméthylammonium, les 2-alkyl-3-carboxyméthyl-3-hydroxyéthyl imidazolines ayant chacune d'environ 8 à 18 atomes de C dans le groupement alkyle ou acyle, le glycinate de cocoacyl aminoéthyl hydroxyéthyl carboxyméthyle, les composés connus sous le nom INCI de cocamidopropylbétaïne, les composés connus sous le nom INCI de cocoamphodiacétate de disodium.

2. Composition antimicrobienne topique selon la revendication 1, constituée essentiellement d'un dérivé de chitosane quaternisé ayant un motif organique polymérisable et d'un agent tensioactif amphotère.

3. Composition antimicrobienne topique selon la revendication 1, pour une utilisation comme médicament destiné au traitement d'infections de la peau chez les mammifères.

4. Composition antimicrobienne topique selon la revendication 1, pour la préparation d'un shampooing médicamenteux destiné au traitement d'infections microbiennes de la peau.

5. Composition antimicrobienne topique selon l'une quelconque des revendications 1 à 4, comprenant en outre un véhicule choisi dans le groupe constitué par un agent hydratant, un humectant, un agent d'ajustement du pH, un agent déodorant, une fragrance, un agent de conditionnement des cheveux, un agent chélateur, un conservateur, un émulsifiant, un épaississant, un agent solubilisant, un agent tampon, un agent chélateur, un antioxydant, un stabilisant, un améliorateur de pénétration, un anti-irritant, un colorant et un agent tensioactif supplémentaire autre qu'un agent tensioactif amphotère.

6. Composition antimicrobienne topique selon l'une quelconque des revendications 1 à 5, où la concentration en ledit dérivé de chitosane quaternisé utilisé dans ladite composition est comprise entre environ 2 % p/v et 4 % p/v.

7. Composition antimicrobienne topique selon l'une quelconque des revendications 1 à 6, comprenant en outre un agent choisi dans le groupe constitué par un agent protecteur, un émollient, un astringent, un irritant, un kératolytique, un agent d'écran solaire, un agent antibiotique, un agent antifongique, un agent antiviral, un agent antiprotozoaire, un agent anesthésique, un agent anti-inflammatoire stéroïdien, un agent anti-inflammatoire non stéroïdien, un agent antiprurigineux, un agent antioxydant, un agent chimiothérapeutique, un agent antihistaminique, et un agent nettoyant.

8. Composition antimicrobienne topique selon l'une quelconque des revendications 1 à 7, où la composition antimicrobienne topique est efficace dans le traitement d'une infection bactérienne.

9. Composition antimicrobienne topique selon la revendication 8, où l'infection bactérienne est provoquée par un ou plusieurs parmi *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis, Bacillus thuringiensis,* les espèces de *Corynebacterium, Streptomyces lividans, Streptomyces murinus, Streptococcus pyogenes,* l'intertrigo streptococcique, *Streptococcus iniae, Streptococcus pneumoniae, Staphylococcus aureus, Staphylococcus intermedius, Staphylococcus pseudintermedius, Staphylococcus epidermidis, Enterococcus faecium, Salmonella typhi, Escherichia coli, Vibrio cholerae, Neisseria meningitidis, Neisseria gonorrhoeae* et *Pseudomonas aeruginosa.*

10. Composition antimicrobienne topique selon la revendication 9, où l'infection bactérienne est provoquée par *Staphylococcus aureus* ou *Staphylococcus pseudintermedius.*

11. Composition antimicrobienne topique selon la revendication 9, où l'infection bactérienne est provoquée par *Escherichia coli.*

12. Composition antimicrobienne topique selon l'une quelconque des revendications 5 à 11, où l'agent chélateur est choisi dans le groupe constitué par l'acide lactique, l'acide tartrique, l'acide adipique, l'acide succinique, l'acide citrique, l'acide ascorbique, l'acide malique, l'acide mandélique, l'acide acétique, l'acide sorbique, le pyrophosphate acide de sodium, l'hexamétaphosphate acide de sodium et l'acide éthylènediaminetétraacétique, et des sels de ceux-ci.

13. Composition antimicrobienne topique selon l'une quelconque des revendications 5 à 12, où le stabilisant est choisi dans le groupe constitué par le monostéarate de glycérol, l'acide stéarique, la triéthanolamine, l'éthanol, le polysorbate 20, l'alcool cétylique, l'alcool stéarylique, le bromure de cétrimonium, l'acide citrique, la cyclométhicone, la diméthicone, le céteth-20, le cétéareth-20, les triglycérides capryliques/ capriques, le polyhydroxystéarate de PEG-40, la polyvinylpyrrolidone, l'acetum, le stéarate de glycérol, la gomme xanthane, l'huile de géranium, l'huile de lavande, l'huile d'eucalyptus, l'huile de Mélaleuca, l'huile de citron, l'huile d'anis, le DEA-phosphate de cétyle, le stéarate de sodium, le stéarate de potassium, les alcools de laine, le stéarate d'octyle, la cire de carnauba, l'ozokérite, un carbomère, le phénoxyéthanol, le p-hydroxybenzoate de méthyle et le p-hydroxybenzoate de propyle, et des mélanges de ceux-ci.

14. Méthode de préparation d'une composition antimicrobienne topique comprenant un dérivé de chitosane quaternisé ayant des motifs organiques polymérisables et un agent tensioactif amphotère telle que définie selon la revendication 1, comprenant l'étape consistant à mélanger au moins un dérivé de chitosane quaternisé ayant un motif organique polymérisable avec au moins un agent tensioactif amphotère.

15. Composition topique selon l'une quelconque des revendications 1 à 12, pour une utilisation dans une méthode destinée au traitement d'infections de la peau chez les mammifères, comprenant l'étape consistant à appliquer, par voie topique à ladite peau, une quantité efficace de la composition topique.

16. Composition topique selon l'une quelconque des revendications 1 à 12, pour une utilisation dans une méthode destinée au traitement ou à la prévention d'une infection microbienne de la peau chez un mammifère, comprenant l'étape consistant à appliquer la composition topique audit mammifère.

17. Composition topique pour une utilisation selon la revendication 15 ou 16, où ladite composition antimicrobienne topique est appliquée à une peau infectée dudit mammifère.
